# EUROPÄISCHE PATENTSCHRIFT

(11) **EP 1 104 397 B1**
(45) Veröffentlichungstag und Bekanntmachung des Hinweises auf die Patenterteilung: **03.03.2004**
(21) Anmeldenummer: 99940069.0
(22) Anmeldetag: 28.07.1999
(51) Int. Cl.: C07C 67/343, C07C 69/618

(54) **VERFAHREN ZUR HERSTELLUNG VON ATROPASÄUREETHYLESTER**
METHOD FOR PRODUCING ATROPIC ACID ETHYL ESTER
PROCEDE PERMETTANT DE FABRIQUER UN ETHYLESTER D'ACIDE ATROPIQUE

(30) Priorität: 11.08.1998 DE 19836222
(43) Veröffentlichungstag der Anmeldung: 06.06.2001
(73) Patentinhaber: BASF AKTIENGESELLSCHAFT, 67056 Ludwigshafen (DE)
(72) Erfinder: FALKENBERG, Wolfgang, D-67368 Westheim (DE); THYES, Marco, D-67061 Ludwigshafen (DE); WENZ, Conny, D-67161 Gönnheim (DE); SCHNEIDER, Ulrich, D-66424 Homburg (DE)
(74) Vertreter: Dörper, Thomas Michael, Dr.
(86) Internationale Anmeldenummer: PCT/EP1999/005403
(87) Internationale Veröffentlichungsnummer: WO 2000/009469

(56) Entgegenhaltungen:
- EP-A- 0 003 670
- DE-A- 3 317 356

## Beschreibung

Die vorliegende Erfindung betrifft ein Verfahren zur Herstellung von Atropasäureethylester.

Atropasäureethylester ist ein wichtiges Zwischenprodukt zur Herstellung pharmazeutischer Wirkstoffe wie Bicyclophenamin, Bornaprin und Tilidin. Das Opioidanalgetikum Tilidin kann beispielsweise durch Umsetzung von Atropasäureethylester mit Dimethylaminobutadien hergestellt werden. Für diese Synthese ist es wichtig, daß der als Ausgangsmaterial verwendete Atropasäureethylester möglichst frei von Atropasäuremethylester ist, da eine solche Verunreinigung dazu führt, daß das Tilidin mit dem entsprechenden Methylester verunreinigt ist, der nur mit einem unverhältnismäßig großem Aufwand entfernt werden kann. Es ist deshalb für eine brauchbare Tilidin-Synthese erforderlich, einen Atropasäureethylester als Ausgangsmaterial zu verwenden, dessen Gehalt an Methylester bei maximal 0,05 % liegt.

Atropasäureethylester mit einem Gehalt an Atropasäuremethylester von maximal 0,05 % kann in einem relativ umständlichen, zweistufigen Verfahren (Helv. Chim. Acta 30, 1349 (1947)) erhalten werden, sofern die Einsatzstoffe nicht mehr als 0,05 % an Methylverbindungen enthalten. Nach diesem Verfahren wird Phenylessigsäureethylester mit Diethyloxalat in Gegenwart von Natriumethanolat zu Diethyl-2-oxo-3-phenylsuccinat, Na-Salz kondensiert. Durch anschließende Umsetzung mit Formaldehyd in Anwesenheit von Kaliumcarbonat wird der Atropasäureethylester mit einem Gehalt an Methylester von maximal 0,05 % erhalten.

In DE 33 17 356 wird ein einstufiges Verfahren beschrieben, in dem Phenylessigsäureethylester mit Paraformaldehyd in Gegenwart von Kaliumcarbonat in einer fest-flüssig phasentransferkatalysierten Reaktion zu Atropasäureethylester umgesetzt wird. Jedoch entstehen hier auch bei Einsatz von Phenylessigsäureethylester mit einem Methylestergehalt von 0,01 % unakzeptabel hohe Mengen an Atropasäuremethylester, wahrscheinlich als Folge der Bildung von Methanol durch eine Cannizzaro-Reaktion von Formaldehyd. So werden bei Verwendung von Toluol als Lösungsmittel etwa 2 % des Methylesters erhalten.

In EP-A 3 670 wird ein einstufiges Verfahren zur Herstellung von 2-Arylacrylsäureestern beschrieben, in dem Arylessigsäureester in einem polaren Solvens in Gegenwart einer Base mit Formaldehyd zu 2-Arylacrylsäureestern umgesetzt werden. Setzt man nach der allgemeinen Verfahrensvorschrift jedoch Phenylessigsäureethylester mit Paraformaldehyd um, so erhält man ein Reaktionsgemisch, welches nur zu etwa 25 % aus Atropasäureethylester besteht.

Es wurde nun ein brauchbares und einfach durchführbares Verfahren zur Herstellung von Atropasäureethylester mit einem Gehalt an Methylester von maximal 0,05 % gefunden.

Gegenstand der Erfindung ist ein Verfahren zur Herstellung von Atropasäureethylester mit einem Methylester-Gehalt von maximal 0,05 % durch Umsetzung von Phenylessigsäureethylester mit Paraformaldehyd in Gegenwart einer Base, dadurch gekennzeichnet, daß man als Phenylessigsäureethylester ein Produkt einsetzt, welches höchstens 0,03 % des entsprechenden Phenylessigsäuremethylesters enthält, und daß man die Reaktion mit nur einer Base in N-Methylpyrrolidon oder N,N-Dimethylformamid durchführt.

In dem Verfahren wird 1 Mol Phenylessigsäureethylester mit 1 bis 2, vorzugsweise 1,4 bis 1,6 Äquivalenten Paraformaldehyd umgesetzt.

Als Base wird in der Regel Kaliumkarbonat verwendet, welches in einer Menge von 1 bis 2, vorzugsweise 1,4 bis 1,8 Mol pro Mol Phenylessigsäureethylester eingesetzt wird.

Die Reaktion wird bei 70 bis 90°C durchgeführt. Sie ist in der Regel nach 1,5 bis 3 h beendet.

Das erfindungsgemäße Verfahren liefert den Atropasäureethylester in Ausbeuten von über 50 % in hoher Produkt-Reinheit (über 90 % (GC)). Der Methylestergehalt des nach dem neuen Verfahren hergestellten Atropasäureethylesters liegt im Bereich von 0,02 bis 0,05 %.

Im Gegensatz zu dem zweistufigen Verfahren gemäß Helv. Chim. Acta 30, 1349 (1947) entfällt bei dem neuen Verfahren die Isolierung und Lagerung einer Zwischenstufe. Das neue Verfahren kann zudem in wesentlich kürzerer Zeit durchgeführt werden. Schließlich benötigt man für das neue Verfahren nicht das nur zeitlich begrenzt lagerfähige und ätzende Natriummethylat.

### Beispiel

94 g Phenylessigsäureethylester (Methylester-Gehalt 0,01 %) wurden in 300 ml N-Methylpyrrolidon mit 120 g Kaliumcarbonat und 25 g Paraformaldehyd bei 75 bis 80°C 1,5 h gerührt. Nach dem Abkühlen wurden 150 ml Wasser zugegeben und die wässrige Phase abgetrennt. Die N-Methylpyrrolidon-Phase wurde zweimal mit je 75 ml Diisopropylether extrahiert. Die vereinigten Diisopropylether-Phasen wurden mit 50 ml Wasser gewaschen und im Vakuum eingeengt. Es wurden 58 g Produkt entsprechend 54 g Atropasäureethylester (53 % der Theorie) mit einem Gehalt an Atropasäuremethylester von 0,03 % erhalten.

## Patentansprüche

1. Verfahren zur Herstellung von Atropasäureethylester mit einem Methylester-Gehalt von maximal 0,05 % durch Umsetzung von Phenylessigsäureethylester mit Paraformaldehyd in Gegenwart einer Base, **dadurch gekennzeichnet, dass** man als Phenylessigsäureethylester ein Produkt einsetzt, welches höchstens 0,03 % des entsprechenden Phenylessigsäuremethylesters enthält, und dass man die Reaktion mit nur einer Base in N-Methylpyrrolidon oder N,N-Dimethylformamid durchführt.

2. Verfahren nach Anspruch 1, **dadurch gekennzeichnet, dass** man als Base Kaliumcarbonat einsetzt.

3. Verfahren nach Anspruch 1 und 2, **dadurch gekennzeichnet, dass** man die Umsetzung in N,N-Dimethylformamid durchführt.

4. Verfahren nach den Ansprüchen 1 bis 3, **dadurch gekennzeichnet, dass** man bezogen auf die Menge an umzusetzendem Phenylessigsäureethylester 1 bis 2 Moläquivalente K₂Co₃ einsetzt.

5. Verfahren nach den Ansprüchen 1 bis 4, **dadurch gekennzeichnet, dass** man bezogen auf die Menge an umzusetzendem Phenylessigsäureethylester 1,4 bis 1,8 Moläquivalente K₂Co₃ einsetzt.

6. Verfahren nach den Ansprüchen 1 bis 5, **dadurch gekennzeichnet, dass** man die Umsetzung bei einer Temperatur von 70 bis 90°C durchführt.

7. Verfahren nach den Ansprüchen 1 bis 6, **dadurch gekennzeichnet, dass** man die Umsetzung nach 1,5 bis 3 h beendet.

8. Verfahren nach den Ansprüchen 1 bis 7, **dadurch gekennzeichnet, dass** man bezogen auf die Menge an umzusetzendem Phenylessigsäureethylester 1 bis 2 Moläquivalente Paraformaldehyd einsetzt.

## Claims

1. A process for preparing ethyl atropate with a methyl ester content not exceeding 0.05% by reacting ethyl phenylacetate with paraformaldehyde in the presence of a base, wherein the product employed as ethyl phenylacetate contains not more than 0.03% of the corresponding methyl phenylacetate, and wherein the reaction is carried out with only one base in N-methylpyrrolidone or N,N-dimethylformamide.

2. A process as claimed in claim 1, wherein potassium carbonate is employed as base.

3. A process as claimed in claim 1 and 2, wherein the reaction is carried out in N,N-dimethylformamide.

4. A process as claimed in claims 1 to 3, wherein from 1 to 2 mole equivalents of K₂CO₃ are employed, based on the amount of ethyl phenylacetate to be reacted.

5. A process as claimed in claims 1 to 4, wherein 1.4 to 1.8 mole equivalents of K₂CO₃ are employed, based on the amount of ethyl phenylacetate to be reacted.

6. A process as claimed in claims 1 to 5, wherein the reaction is carried out at a temperature of from 70 to 90°C.

7. A process as claimed in claims 1 to 6, wherein the reaction is terminated after 1.5 to 3 h.

8. A process as claimed in claims 1 to 7, wherein from 1 to 2 mole equivalents of paraformaldehyde are employed, based on the amount of ethyl phenylacetate to be reacted.

## Revendications

1. Procédé de préparation d'éthylesters d'acide atropique présentant une teneur en méthylester d'un maximum de 0,05% en poids, par réaction d'un éthylester d'acide phénylacétique avec du paraformaldéhyde en présence d'une base, **caractérisé en ce que** l'on met en oeuvre comme éthylester d'acide phénylacétique un produit qui contient au maximum 0,03% du méthylester d'acide phénylacétique correspondant, et que l'on entreprend la réaction avec seulement une base dans de la N-méthylpyrrolidone ou du N,N-diméthylformamide.

2. Procédé selon la revendication 1, **caractérisé en ce que** l'on utilise comme base du carbonate de potassium.

3. Procédé selon les revendications 1 et 2, **caractérisé en ce que** l'on entreprend la réaction dans du N,N-diméthylformamide.

4. Procédé selon les revendications 1 à 3, **caractérisé en ce que** l'on met en oeuvre, par rapport à la quantité d'éthylester d'acide phénylacétique à convertir, de 1 à 2 équivalents molaires de K₂CO₃.

5. Procédé selon les revendications 1 à 4, **caractérisé en ce que** l'on met en oeuvre, par rapport à la quantité d'éthylester d'acide phénylacétique à convertir, de 1,4 à 1,8 équivalents molaires de K₂CO₃.

6. Procédé selon les revendications 1 à 5, **caractérisé en ce que** l'on entreprend la réaction à une température de 70 à 90°C.

7. Procédé selon les revendications 1 à 6, **caractérisé en ce que** l'on termine la réaction après 1,5 à 3 h.

8. Procédé selon les revendications 1 à 7, **caractérisé en ce que** l'on met en oeuvre, par rapport à la quantité d'éthylester d'acide phénylacétique à convertir, de 1 à 2 équivalents molaires de paraformaldéhyde.
